# EUROPEAN PATENT SPECIFICATION

(11) **EP 4 527 322 B1**
(45) Date of publication and mention of the grant of the patent: **14.10.2026**
(21) Application number: 25156854.9
(22) Date of filing: 23.12.2019
(51) Int. Cl.: A61B 17/072

(54) **APPLICATOR FOR SURGICAL STAPLER BUTTRESS**
APPLIKATOR FÜR CHIRURGISCHE KLAMMERVERSTEIFUNG
APPLICATEUR POUR CONTREFORT D'AGRAFEUSE CHIRURGICALE

(30) Priority: 28.12.2018 US 201816235522
(43) Date of publication of application: 26.03.2025
(62) Document number(s) of the earlier application(s) in accordance with Art. 76 EPC: 19219463.7 / 3 673 827
(73) Proprietor: Ethicon LLC, 00969 Guaynabo (PR)
(72) Inventor: Vendely, Michael J., Cincinnati, 45242 (US); Barton, Trevor J., Cincinnati, 45242 (US); Schellin, Emily A., Cincinnati, 45242 (US); Zeiner, Mark S., Cincinnati, 45242 (US)
(74) Representative: Carpmaels & Ransford LLP

(56) References cited:
- US-A1- 2017 055 981

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through the cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasonic vibration, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; and U.S. Pat. No. 8,453,914, entitled "Motor-Driven Surgical Cutting Instrument with Electric Actuator Directional Control Assembly," issued June 4, 2013.

While the surgical staplers referred to above are described as being used in endoscopic procedures, it should be understood that such surgical staplers may also be used in open procedures and/or other non-endoscopic procedures. By way of example only, a surgical stapler may be inserted through a thoracotomy, and thereby between a patient's ribs, to reach one or more organs in a thoracic surgical procedure that does not use a trocar as a conduit for the stapler. Such procedures may include the use of the stapler to sever and close a vessel leading to a lung. For instance, the vessels leading to an organ may be severed and closed by a stapler before removal of the organ from the thoracic cavity. Of course, surgical staplers may be used in various other settings and procedures.

Examples of surgical staplers that may be particularly suited for use through a thoracotomy are disclosed in U.S. Pat. No. 9,186,142, entitled "Surgical Instrument End Effector Articulation Drive with Pinion and Opposing Racks," issued November 17, 2015; U.S. Pat. No. 9,717,497, entitled "Lockout Feature for Movable Cutting Member of Surgical Instrument," issued August 1, 2017; U.S. Pat. No. 9,517,065, entitled "Integrated Tissue Positioning and Jaw Alignment Features for Surgical Stapler," issued December 13, 2016; U.S. Pat. No. 9,839,421, entitled "Jaw Closure Feature for End Effector of Surgical Instrument," issued December 12, 2017; U.S. Pat. No. 9,867,615, entitled "Surgical Instrument with Articulation Lock having a Detenting Binary Spring," issued January 16, 2018; U.S. Pat. No. 9,622,746, entitled "Distal Tip Features for End Effector of Surgical Instrument," issued April 18, 2017; U.S. Pat. Pub. No. 2014/0239037, entitled "Staple Forming Features for Surgical Stapling Instrument," published August 28, 2014; U.S. Pat. No. 9,795,379, entitled "Surgical Instrument with Multi-Diameter Shaft," issued October 24, 2017; and U.S. Pat. No. 9,808,248, entitled "Installation Features for Surgical Instrument End Effector Cartridge," issued November 7, 2017.

In some instances, it may be desirable to equip a surgical stapling instrument with a buttress material to reinforce the mechanical fastening of tissue provided by staples. Such a buttress may prevent the applied staples from pulling through tissue and may otherwise reduce a risk of tissue tearing at or near the site of applied staples. Such buttress material may be applied to the surgical stapling instrument with a buttress applier cartridge. The buttress applier cartridge retains the buttress material prior to application and releases the buttress material once applied to the surgical stapling instrument. An example of such buttress applier cartridge is disclosed in U.S. Pat. Pub. No. 2017/0056016, entitled "Surgical Stapler Buttress Applicator with End Effector Actuated Release Mechanism," published March 2, 2017.

US 2017/055981 describes a buttress applied to an end effector of a surgical stapler. The buttress is loaded on a platform of a buttress applier cartridge, and secured to the end effector by an adhesive layer.

While various kinds of surgical stapling instruments and associated components have been made and used, it is believed that no one prior to the inventor(s) has made or used the invention described in the appended claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary buttress applier cartridge assembly that includes an example of a buttress applier cartridge carrying an example of a buttress assembly for an upper jaw and an example of another buttress assembly for a lower jaw;
FIG. 2 depicts an exploded perspective view of the buttress applier cartridge assembly of FIG. 1 including a chassis and a platform in addition to a pair of buttress assemblies;
FIG. 3 depicts a perspective view of the chassis with the platform of FIG. 2;
FIG. 4 depicts a front perspective view of the platform of FIG. 2;
FIG. 5 depicts a rear perspective view of the platform of FIG. 2;
FIG. 6 depicts an elevational side view of the platform of FIG. 2;
FIG. 7 depicts a top perspective view of a left actuator sled of the buttress applier cartridge assembly of FIG. 2;
FIG. 8 depicts a bottom perspective view of the left actuator sled of FIG. 7;
FIG. 9 depicts a top perspective view of a right actuator sled of the buttress applier cartridge assembly of FIG. 2;
FIG. 10 depicts a bottom perspective view of the right actuator sled of FIG. 9;
FIG. 11 depicts a perspective view of the chassis and the platform of FIG. 3 with pairs of left and right actuator sleds of FIG. 7 and FIG. 9;
FIG. 12 depicts a cross-sectional view of the chassis, the platform, and the actuator sleds of FIG. 11 taken along section line 12-12 of FIG. 11;
FIG. 13A depicts a top view of the chassis, the platform, and the actuator sleds of FIG. 11 in a restraint position;
FIG. 13B depicts the top view of the chassis, the platform, and the actuator sleds similar to FIG. 13A, but showing the actuator sleds being directed from the restraint position toward a release position;
FIG. 13C depicts the top view of the chassis, the platform, and the actuator sleds similar to FIG. 13B, but showing the actuator sleds in the release position;
FIG. 14A depicts a perspective view of an end effector of an exemplary surgical instrument showing the buttress applier cartridge assembly of FIG. 1 approaching the end effector with the upper and lower jaws in an open position;
FIG. 14B depicts the perspective view of the end effector similar to FIG. 14A, but showing the buttress applier cartridge assembly of FIG. 1 positioned between the upper and lower jaws in a closed position;
FIG. 15 depicts a side sectional view of the end effector and the platform of the buttress applier cartridge of FIG. 14B in an exemplary parallel-camber orientation, but with various features hidden for greater clarity;
FIG. 16 depicts a perspective view of the end effector similar to FIG. 14B, but showing the buttress assemblies respectively secured to the upper and lower jaws in the open position and the buttress applier cartridge removed therefrom;
FIG. 17A depicts a sectional side view of a portion of the end effector of FIG. 14B with the buttress assemblies of FIG. 1 applied to the end effector and tissue positioned between the buttress assemblies with the upper and lower jaws in the open position;
FIG. 17B depicts the sectional side view of the portion of the end effector and the buttress assemblies similar to FIG. 17A, but showing the upper and lower jaws in the closed position;
FIG. 17C depicts the sectional side view of the buttress assemblies similar to FIG. 17B, but showing the buttress assemblies secured to the tissue with a staple formed in the tissue;
FIG. 18 depicts a perspective view of staples and the buttress assembly of FIG. 17C having been secured to the tissue by the end effector and cut by a knife;
FIG. 19 depicts the side sectional view of the end effector and the platform of the buttress applier cartridge similar to FIG. 15, but with the end effector in an exemplary over-camber orientation; and
FIG. 20 depicts the side sectional view of the end effector and the platform of the buttress applier cartridge similar to FIG. 15, but with the end effector in an exemplary under-camber orientation.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. The invention is defined by the independent claims. Optional features are recited in the dependent claims. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It should be understood that terms such as "proximal" and "distal" are used herein with reference to a clinician gripping a surgical instrument, such as surgical and severing instrument (210) and buttress applier cartridge assembly (10) discussed below. It will be further appreciated that for convenience and clarity, spatial terms such as "left," "right," "front," "rear," "upright," "upside-down," "upper," "lower," "bottom," and "top" are used herein with respect to the drawings. However, surgical instruments are used in many orientations and positions, and these terms are not intended to be limiting and absolute.

### I. Exemplary Buttress Applier Cartridge Assembly

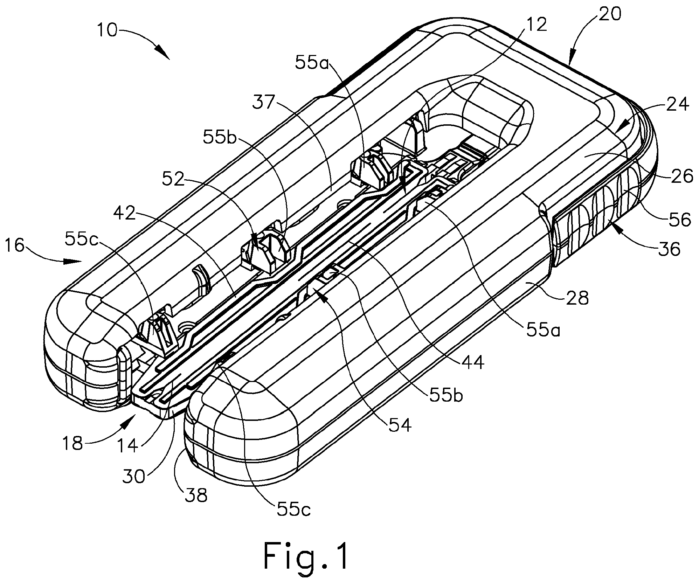

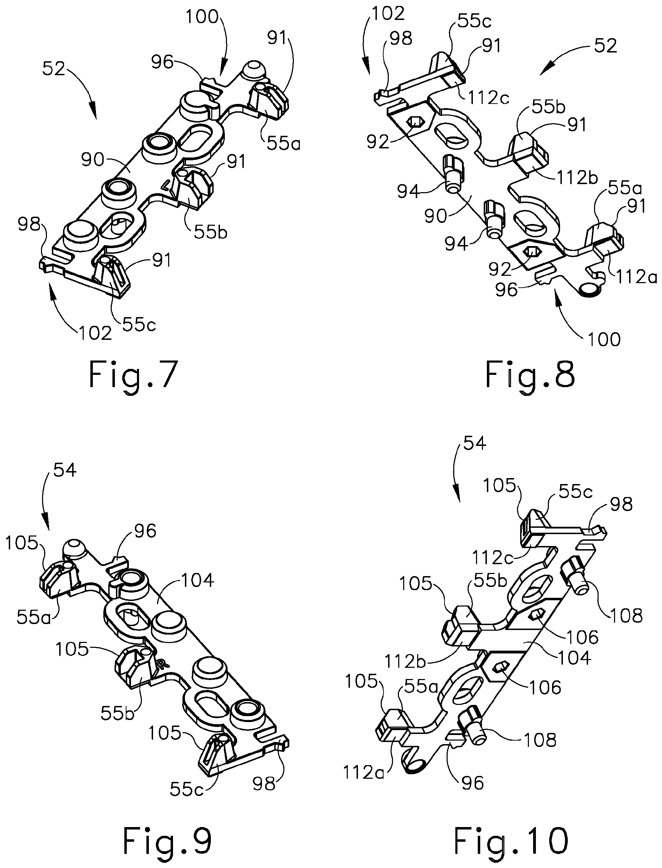

In some instances, it may be desirable to use an exemplary buttress applier cartridge assembly (10) as shown in FIG. 1 to equip a surgical instrument with a buttress assembly (12) for forming staples in tissue with a buttress (14). Such buttress (14) inhibits the formed staples from pulling through the tissue to thereby reduce a risk of tissue tearing at or near the site of formed staples. In addition to or as an alternative to providing structural support and integrity to a line of staples, buttress (14) may provide various other kinds of effects such as spacing or gap-filling, administration of therapeutic agents, and/or other effects. Prior to use with the surgical instrument, one or more buttresses (14) is releasably retained on a buttress applier cartridge (16), which is configured to deposit buttress assembly (10) onto surgical instrument for use as discussed below in more detail in an exemplary surgical instrument (18) *(see* FIG. 10A).

FIG. 1 shows buttress applier cartridge assembly (10) including a pair of buttress assemblies (12) releasably retained on buttress applier cartridge (16), which supports and protects buttress assemblies (12) prior to use and further aids with loading buttress assemblies (12) on surgical instrument (210) *(see* FIG. 10A). Buttress applier cartridge (16) of the present example includes an open end (18) and a closed end (20). Open end (18) is configured to receive end effector (212) *(see* FIG. 10A) as described below in greater detail. Buttress applier cartridge (16) further includes a housing assembly (24) having an upper housing (26) and a lower housing (28), which each generally define a "U" shape to present open end (18). Various components are interposed between upper and lower housings (26, 28). In particular, these components include a platform (30) supporting a chassis (36).

Platform (30) of the present example supports upper buttress assembly (12) on one side of platform (30) and lower buttress assembly (12) on the other side of platform (30). Platform (30) is exposed in recesses that are formed between the prongs of the "U" configuration of upper and lower housings (26, 28). Thus, upper housing (26) has an upper gap (37) extending to the open end (18) along an upper surface of platform (30), and lower housing (28) similarly has a lower gap (38) extending to the open end (18) along the lower surface of platform (30). The location of platform (30) and buttress assemblies (12) in such recesses may prevent inadvertent contact between buttress assemblies (12) and other devices in the operating room. In other words, upper and lower housings (26, 28) may provide some degree of physical shielding of buttress assemblies (12) while buttress assemblies are retained on platform (30).

Additional features may be combined as applicable with the following example of buttress applier cartridge assembly (10). Such features are described in U.S. Pat. App. No. 2020/0205820, entitled "Adhesive Distribution on Buttress for Surgical Stapler," filed on even date herewith; U.S. Pat. App. No. 2020/0205822, entitled "Surgical Stapler Buttress with Tissue In-Growth Promotion," filed on even date herewith; U.S. Pat. App. No. 2020/0205821, entitled "Configuration of Buttress for Surgical Stapler," filed on even date herewith; U.S. Pat. App. No. 2020/0205824, entitled "Packaging for Surgical Stapler Buttress," filed on even date herewith; U.S. Pat. App. No. 2020/0205825, entitled "Method of Applying Buttresses to Surgically Cut and Stapled Sites," filed on even date herewith; U.S. Pat. App. No. 2020/0205826, entitled "Curved Tip Surgical Stapler Buttress Assembly Applicator with Opening Feature for Curved Tip Alignment," filed on even date herewith; U.S. Pat. App. No. 2020/0205827, entitled "Curved Tip Surgical Stapler Buttress Assembly Applicator with Proximal Alignment Features," filed on even date herewith; and U.S. Pat. App. No. 2020/0205807, entitled "Curved Tip Surgical Stapler Buttress Assembly Applicator with Compression Layer Pocket Feature," filed on even date herewith; U.S. Patent App. No. D901,686, entitled "Applicator for a Surgical Stapler Buttress," filed on even date herewith; U.S. Patent App. No. D933,220, entitled "Buttress for Surgical Stapler," filed on even date herewith; U.S. Patent App. No. D922,576, entitled "Tray for Surgical Stapler Buttress Applicator," filed on even date herewith; U.S. Pat. App. No. D903,115, entitled "Applicator for a Surgical Stapler Buttress," filed on even date herewith; and U.S. Pat. App. No. D932,621, entitled "Buttress Assembly for a Surgical Stapler," filed on even date herewith.

### A. Exemplary Buttress Assembly

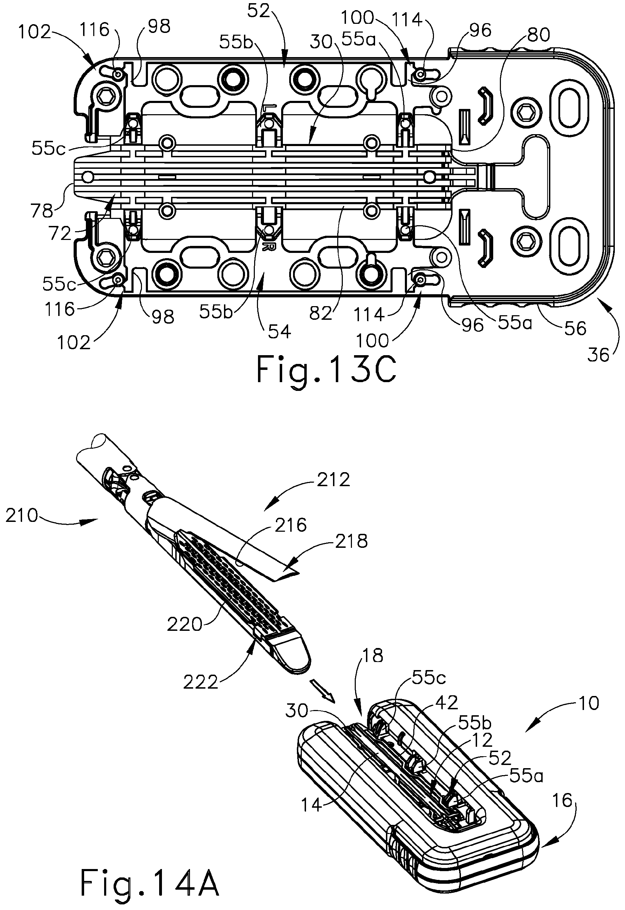

With respect to FIG. 1, upper and lower buttress assemblies (12) are structurally identical, but for the relative positions of upper and lower buttress assemblies (12) retained on buttress applier cartridge (16). Buttress applier cartridge assembly (10) may thus be used in more than one orientation with surgical instrument (210) *(see* FIG. 14A). It will be appreciated that the following description of upper buttress assembly (12) similarly applies to lower buttress assembly (12) but for the respective orientations.

Upper buttress assembly (12) includes buttress (14) and an upper adhesive layer (42). Buttress (14) of the present example more particularly has a three-layer, polymer construction including a core layer sandwiched between two outer layers to be collectively strong yet flexible to support a line of staples. In the present example, core layer is a polyglactin 910 material, which is manufactured and sold by Ethicon, Inc. of Somerville, New Jersey as VICRYL, whereas each outer layer is a polydioxanone (PDO) film material. Buttress (14) of the present example is formed by laminating core layer between outer layers under a predetermined pressure, a predetermined temperature, and a predetermine time. Buttress (14) is further mechanically cut to size thereby inhibiting abrasive edges, such as burrs and/or delamination, that could damage sensitive tissues. It will be appreciated that alternative methods of cutting buttresses (14), such as a laser cutting or hot knife cutting, may be similarly used.

By way of further example only, each buttress (14) may comprise one or more of the following: NEOVEIL absorbable PGA felt by Gunze Limited, of Kyoto, Japan; SEAMGUARD polyglycolic acid: trimethylene carbonate (PGA:TMC) reinforcement material by W.L. Gore & Associates, Inc., of Flagstaff, Arizona; PERI-STRIPS DRY with VERITAS Collagen Matrix (PSDV) reinforcement material, by Baxter Healthcare Corporation of Deerfield, Illinois; BIODESIGN biologic graft material by Cook Medical, Bloomington, Indiana; and/or SURGICEL NU-KNIT hemostat material by Ethicon, Inc. of Somerville, New Jersey. Still other suitable materials that may be used to form each buttress (14) will be apparent to those of ordinary skill in the art in view of the teachings herein.

In addition or in the alternative, each buttress (14) may comprise a material including, for example, a hemostatic agent such as fibrin to assist in coagulating blood and reduce bleeding at the severed and/or stapled surgical site along tissue. As another merely illustrative example, each buttress (14) may comprise other adjuncts or hemostatic agents such as thrombin may be used such that each buttress (14) may assist to coagulate blood and reduce the amount of bleeding at the surgical site. Other adjuncts or reagents that may be incorporated into each buttress (14) may further include but are not limited to medical fluid or matrix components. Merely illustrative examples of materials that may be used to form each buttress (14), as well as materials that may be otherwise incorporated into each buttress (14), are disclosed in U.S. Patent App. No. 14/667,842, entitled "Method of Applying a Buttress to a Surgical Stapler," filed March 25, 2015. Alternatively, any other suitable materials may be used.

By way of further example only, each buttress (14) may be constructed in accordance with at least some of the teachings of U.S. Pat. Pub. No. 2012/0241493, entitled "Tissue Thickness Compensator Comprising Controlled Release and Expansion," published September 27, 2012; U.S. Pat. Pub. No. 2013/0068816, entitled "Surgical Instrument and Buttress Material," published March 21, 2013; U.S. Pat. No. 9,999,408, entitled "Surgical Instrument with Fluid Fillable Buttress," issued June 19, 2018; U.S. Pat. No. 8,814,025, entitled "Fibrin Pad Matrix with Suspended Heat Activated Beads of Adhesive," issued August 26, 2014; U.S. Pat. No. 8,899,464, entitled "Attachment of Surgical Staple Buttress to Cartridge," issued December 2, 2014; U.S. Pat. No. 9,492,170, entitled "Device for Applying Adjunct in Endoscopic Procedure," issued November 15, 2016; U.S. Pat. No. 8,998,060, entitled "Resistive Heated Surgical Staple Cartridge with Phase Change Sealant," issued April 7, 2015; U.S. Pat. No. 9,393,018, entitled "Surgical Staple Assembly with Hemostatic Feature," issued July 19, 2016; U.S. Pat. No. 9,101,359, entitled "Surgical Staple Cartridge with Self-Dispensing Staple Buttress," issued August 11, 2015; U.S. Pat. No. 9,198,644, entitled "Anvil Cartridge for Surgical Fastening Device," issued December 1, 2015; U.S. Pat. Pub. No. 2013/0075447, entitled "Adjunct Therapy for Applying Hemostatic Agent," published March 28, 2013; U.S. Pat. No. 9,211,120, entitled "Tissue Thickness Compensator Comprising a Plurality of Medicaments," issued December 15, 2015; U.S. Pat. Pub. No. 2015/0351758, entitled "Adjunct Materials and Methods of Using Same in Surgical Methods for Tissue Sealing," published December 10, 2015; U.S. Pat. Pub. No. 2017/0049444, entitled "Implantable Layers for a Surgical Instrument," published February 23, 2017; U.S. Pat. Pub. No. 2017/0055986, entitled "Drug Eluting Adjuncts and Methods of Using Drug Eluting Adjuncts," published March 2, 2017; U.S. Pat. Pub. No. 2017/0086837, entitled "Compressible Adjunct with Crossing Spacer Fibers," published March 30, 2017; and/or U.S. Pat. Pub. No. 2017/0086842, entitled "Method for Applying an Implantable Layer to a Fastener Cartridge," published March 30, 2017.

Furthermore, buttress (14) is configured to be cut by a knife (not shown) from a proximal portion of buttress (14), along an intermediate portion of buttress (14), and further through a distal portion of buttress (14) such that inward edges are adjacent to cut tissue as discussed below in more detail. Buttress (14) further includes a longitudinally extending pre-cut slit (44) configured to receive knife (not shown) and aid in separating lateral portions of buttress (14) as inward edges form therealong.

Upper adhesive layer (42) is provided on outer layer of buttress (14) in order to adhere buttress (14) within effector (212) *(see* FIG. 14A) of surgical instrument (210) *(see* FIG. 14A). Adherence of the buttress (14) can occur through a variety of mechanisms including but not limited to a pressure sensitive adhesive. In the case of pressure sensitive adhesion, adhesion occurs upon the application of at least a predetermined minimum force. In some versions, each adhesive layer (42) includes a pressure sensitive adhesive material. Examples of various suitable materials that may be used to form adhesive layers (42) are disclosed in U.S. Pat. Pub. No. 2016/0278774, entitled "Method of Applying a Buttress to a Surgical Stapler," published September 29, 2016. Alternatively, any other suitable materials may be used. As shown in the present example, adhesive layer (42) is applied to form a continuous outer seal to enhance longevity once applied to end effector (212) *(see* FIG. 14A).

It should be understood that the term "adhesive," as used herein, may include (but is not limited to) tacky materials and also materials that are pliable or wax-like and adhere to a complex geometry via deformation and conformance. Some suitable adhesives may provide such pliability to adhere to a complex geometry via deformation and conformance without necessarily providing a high initial tack. In some instances, adhesives with lower tackiness may be removed more cleanly from surfaces. Various suitable materials that may be used to form adhesive layers (42) will be apparent to those of ordinary skill in the art in view of the teachings herein.

### B. Exemplary Buttress Applier Cartridge

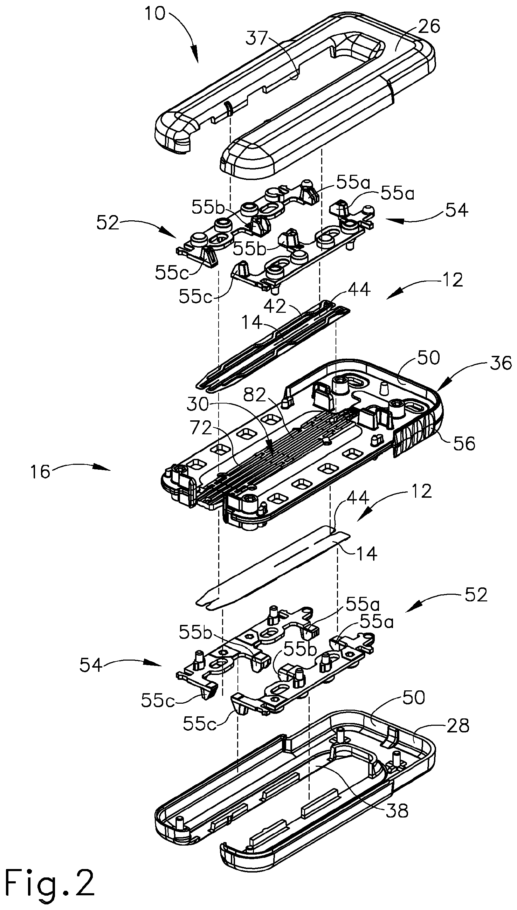

As shown in FIG. 2, buttress applier cartridge (16) includes chassis (36) supporting platform (30) as well as upper and lower housings (26, 28) of housing assembly (24) configured to connect together to define an interior space (50). An upper left actuator sled (52) and an upper right actuator sled (54) are movably connected to an upper face chassis (36) within interior space (50), while a lower left actuator sled (52) and a lower right actuator sled (54) are movably connected to a lower face of chassis (36) within interior space (50). Upper right and left actuator sleds (52, 54) retain upper buttress assembly (12) on platform (30) in a restraint position, but are configured to move from the restraint position to a release position for depositing the upper buttress assembly (12) on end effector *(212) (see* FIG. 14A). Similarly, lower right and left actuator sleds (52, 54) retain lower buttress assembly (12) on platform (30) in the restraint position, but are configured to move from the restraint position to the release position for depositing the lower buttress assembly (12) on end effector (212) *(see* FIG. 14A). In the present example, left actuator sled (52) is distinct from right actuator sled (54) for reasons discussed below in greater detail. Also, upper and lower right actuator sleds (52) are structurally identical to each other, and upper and lower left actuator sleds (54) are structurally identical to each other. Thus, upper and lower actuator sleds (52, 54) are interchangeable in this respect and any discussion contained herein directed to a pair of upper actuator sleds (52, 54) is similarly applicable to a pair of lower actuator sleds (52, 54).

Each actuator sled (52, 54) includes a plurality of arms (55a, 55b, 55c) extending laterally inward to selectively and releasably secure buttress assemblies (12) to platform (30). In particular, FIG. 2 show arms (55a, 55b, 55c) positioned such that buttress assemblies (12) are interposed between the free ends of arms (34) and platform (30). Arms (55a, 55b, 55c) are movable laterally outwardly such that arms (55a, 55b, 55c) disengage buttress assemblies (12) as shown in FIG. 2, thereby enabling buttress assemblies (12) to be removed from platform (30). In the present example, arms (55a, 55b, 55c) are configured to bear against buttress assemblies (12) in the restraint position, thereby pinching buttress assemblies (12) against platform (30). Other suitable ways in which arms (5) may engage buttress assemblies (12) will be apparent to those of ordinary skill in the art in view of the teachings herein.

Chassis (36) is configured to cooperate with upper and lower housings (26, 28) to provide a mechanical ground for moving components of buttress applier cartridge (16) and provide structural support for components of buttress applier cartridge (16). Chassis (36) further includes integral gripping features (56) that are exposed on opposite sides of housing assembly (24). Gripping features (56) have a surface geometry configured to promote an operator's grip of buttress applier cartridge (16) during use of buttress applier cartridge (16). Various suitable configurations that may be used for gripping features (56) will be apparent to those of ordinary skill in the art in view of the teachings herein. Similarly, various surface treatments (e.g., elastomeric material, etc.) that may be applied to gripping features (56) will be apparent to those of ordinary skill in the art in view of the teachings herein.

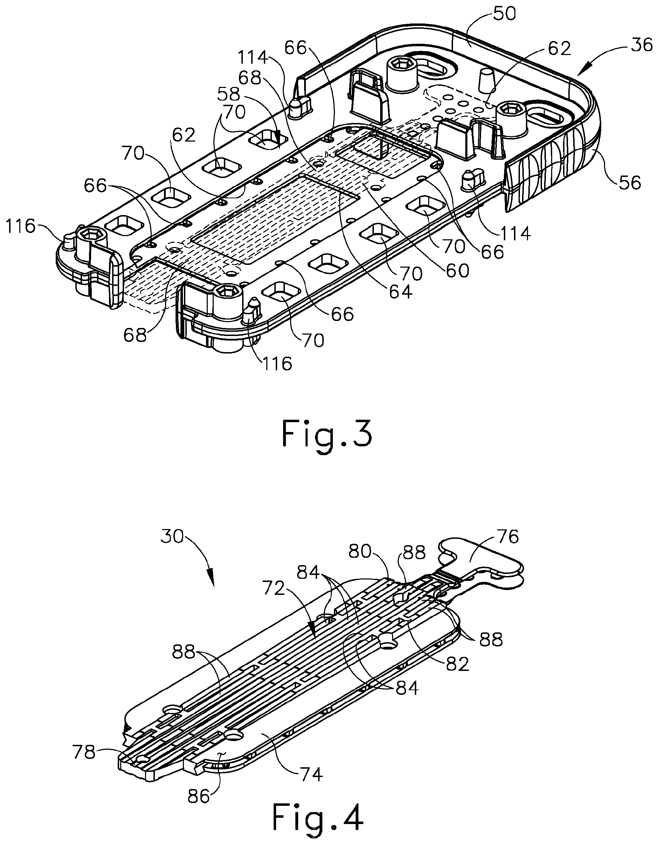

With respect to FIG. 3, platform (30) is connected to and supported by chassis (36) to secure platform (30) relative to upper and lower housing (26, 28) *(see* FIG. 2). In the present example, platform (30) is unitarily formed and molded to a rigid web portion (58) including a frame (60) and defining a plurality of holes (62, 64, 66) configured provide material overlap for mold securement. Holes (62, 64, 66) more particularly include an upper and a lower peripheral recess (62) to the centrally located frame (60). A central slot (64) extends through frame (60) as well as a plurality of through holes (66) spaced laterally about central slot (64). Frame (60) also extends laterally across central slot (64) at bridge portions (68) to provide additional structural rigidity to chassis (36) while providing platform (30) with sufficient clearance for resilient deformation as discussed below in greater detail. Thereby, recess (62), central slot (64), and through holes (66) receive a resilient, elastomeric material to form and secure the material as platform (30) to chassis (36). While the present platform (30) is molded to chassis (36), it will be appreciated that platform (30) may be alternatively secured to chassis (36), and the invention is not intended to be limited to the particular rigid web portion (58) and molding as discussed herein. Various suitable materials and structural configurations that may be used to form platform (30) will be apparent to those of ordinary skill in the art in view of the teachings herein.

Chassis (36) further includes a plurality of sled clearance holes (70) arranged in a pair of rows on opposing lateral sides of chassis (36). Left and right actuator sleds (52, 54) *(see* FIGS. 7-10) connect together on opposing sides of chassis (36) through such sled clearance holes (70) to slide inwardly together in connected pairs. Additional details regarding connection and actuation of left and right actuator sleds (52, 54) *(see* FIGS. 7-10) will be discussed below in greater detail. However, it will be appreciated that any such hole through chassis (36) to provide for fastening clearance of left and right actuator sleds (52, 54) *(see* FIGS. 7-10) may be used, and the invention is not intended to be unnecessarily limited to sled clearance holes (70) as discussed herein.

### i. Exemplary Varying Stiffness Platform for Supporting Buttress Assemblies

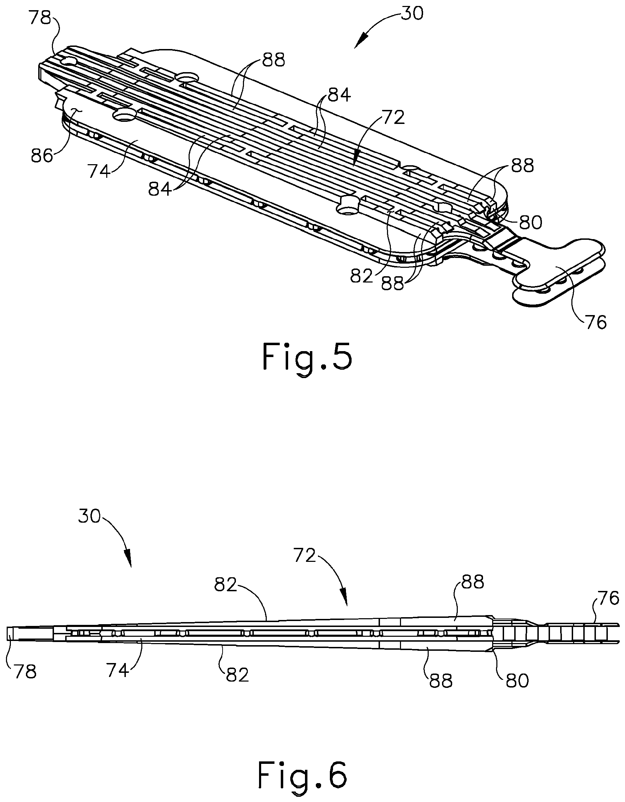

FIGS. 3-6 show one example of platform (30) in additional detail as including a pad (72) having a peripheral securement (74) laterally extending therefrom and an anchor securement (76) distally extending therefrom. Collectively, peripheral and anchor securements (74, 76) are positioned in recesses (62) and extend into through holes (66) to secure pad (72) to chassis (36). In some versions, platform (30) is formed of a material that provides a high coefficient of friction, thereby reducing any tendency that buttress assemblies (12) might otherwise have to slide along corresponding surfaces of platform (30). For instance, platform (30) may comprise a resilient, elastomeric material, such as silicone, to be molded to be formed as both securements (74, 76) and pad (72). One example silicone material is a 30 Durometer, Shore A silicone. To this end, pad (72) is formed with varying stiffness along its longitudinal length to simultaneously provide sufficient reactionary forces of at least the predetermined minimum force for adhesion while accommodating a parallel-camber orientation, an over-camber orientation, and an under-camber orientation of end effector (212) *(see* FIG. 14A) as discussed below in greater detail. As used herein, the term "parallel-camber orientation" refers to an upper jaw and a lower jaw of an end effector being functionally parallel to each other. The term "over-camber orientation" refers to an upper jaw of an end effector being over rotated relative a lower jaw of an end effector. The term "under-camber orientation" refers to an upper jaw being under rotated relative to a lower jaw of an end effector. Exemplary upper and lower jaws will be described in this context below in greater detail.

With respect to FIGS. 4-5, a resilient proximal end (78) pad (72) has a proximal end stiffness and a proximal transverse depth, whereas a resilient distal end (80) of pad (72) has a distal end stiffness and a distal transverse depth. In the present example, proximal end stiffness is generally greater than the distal end stiffness such that initial compression of distal end (80) requires less compressive force than compression of proximal end (78). Of course, further compression of distal end (80) relative to proximal end (78) may result in distal end stiffness increasing to or even exceeding proximal end stiffness so long as the lesser stiffness of distal end (80) is included therein for accommodating the over-cambered orientation of end effector (212) *(see* FIG. 14A).

In addition, distal transverse depth is greater than proximal transverse depth. Thereby, the greater distal transverse depth effectively props up buttress assembly (12) *(see* FIG. 1) for improved engagement with end effector (212) *(see* FIG. 10A) in the under-camber orientation, but the decreased distal end stiffness allows for greater compression to accommodate end effector (212) *(see* FIG. 10A) in the over-camber orientation. Pad (72) of the present example is wedge-shaped having opposing ramp surfaces (82) continuously tapering together from the distal end (80) to the proximal end (78) for accommodating parallel-camber, over-camber, and under-camber orientations along the entire longitudinal length of pad (72). In some examples, depths and stiffnesses along pad (72) are configured to receive a full range of over-camber to under-camber orientations based on determined manufacturing tolerances of end effector (11) *(see* FIG. 10A).

Pad (72) shown in FIGS. 4-6 is unitarily formed of a resilient material having a consistent stiffness throughout. Such longitudinally varying stiffness discussed above is thus generated by forming a plurality of reliefs, such as channels (84), in at least the distal end (80) to reduce the distal end stiffness relative to the proximal end stiffness. In the present example, channels (84), such as five channels (84) are equally spaced laterally apart from each other and longitudinally extend from distal end (80) to proximal end (78). Channels (84) further transversely extend to a base surface (86) common to securements (74, 77) and proximal end (78) to define varying channel depths in the longitudinal direction along pad (72). More particularly, upper channels (84) extend transversely downward from upper ramp surface (82) to upper base surface (86), whereas lower channels (84) extend transversely upward from lower ramp surface (82) to lower base surface (86). In turn, a plurality of ribs (88) are defined between channels (84) and similarly extend from ramp surfaces (82) to base surfaces (86) to support buttress assemblies (12) *(see* FIG. 1) and have varying stiffness from the proximal end (78) to the distal end (80) on each opposing side of pad (72).

### ii. Exemplary Restraint Features for Retention of Buttress Assemblies on Varying Stiffness Platform

FIGS. 2 and 7-10 show restraint features, such as left and right actuator sleds (52, 54) discussed briefly above for releasably securing buttress assemblies (12) to platform (30) in the restraint position. Each of left and right actuator sleds (52, 54) has arms (55a, 55b, 55c) configured to accommodate varying transverse depths along the longitudinal length of pad (72). More particularly, arms (55a, 55b, 55c) include a distal arm (55a), an intermediate arm (55b), and a proximal arm (55c) spaced longitudinally apart from each other and extending laterally inward toward platform (30). Each distal arm (55a), intermediate arm (55b), and proximal arm (55c) of left or right actuator sled (52, 54) transversely extends toward platform (30) such that each of distal arm (55a), intermediate arm (55b), and proximal arm (55c) is offset from the other arms (55a, 55b, 55c) in the transverse direction. Thereby, distal arm (55a), intermediate arm (55b), and proximal arm (55c) are transversely spaced from the ramp surface (82) to trace the contour of the ramp surface (82).

With respect to FIGS. 7-8, upper left actuator sled (52) has a longitudinally extending upper left sled body (90) with distal, intermediate, and proximal arms (55a, 55b, 55c) laterally extending inward toward the right. Each arm (55a, 55b, 55c) of left actuator sled (52) has a cam surface (91) configured to receive end effector (212) *(see* FIG. 14A) thereagainst to urge left actuator sled (52) toward the release position. In addition, a pair of outer dowel holes (92) open downward and are respectively positioned on distal and proximal end portions of upper left sled body (90). A pair of inner dowels (94) extend downward from left sled body (90) between outer dowel holes (92) and in longitudinal alignment with outer dowel holes (92). In order to arrest movement of upper left actuator sled (52) in the restraint and release positions, a distal cantilever catch (96) laterally extends to the left from the distal portion of upper left sled body (90), and a proximal cantilever catch (96) laterally extends to the left from the proximal portion of upper left sled body (90). Distal and proximal cantilever catches (96, 98) are respectively portions of distal and proximal detent couplings (100, 102) discussed below in greater detail.

With respect to FIGS. 9-10, upper right actuator sled (54) has a longitudinally extending upper right sled body (104) with distal, intermediate, and proximal arms (55a, 55b, 55c) laterally extending inward toward the left. Each arm (55a, 55b, 55c) of right actuator sled (54) has a cam surface (105) configured to receive end effector (212) *(see* FIG. 14A) thereagainst to urge left actuator sled (52) toward the release position. In addition, a pair of inner dowel holes (106) open downward and are positioned on upper right sled body (104). A pair of outer dowels (108) extend downward from right sled body (104) and are respectively positioned on distal and proximal end portion of upper right sled body (104) in longitudinal alignment with inner dowel holes (106). In order to arrest movement of upper right actuator sled (54) in the restraint and release positions, another distal cantilever catch (96) laterally extends to the right from the distal portion of upper right sled body (104), and another proximal cantilever catch (96) laterally extends to the right from the proximal portion of upper right sled body (104). Again, distal and proximal cantilever catches (96, 98) are respectively portions of distal and proximal detent couplings (100, 102) discussed below in greater detail.

FIG. 2 and FIGS. 11-12 show upper right and left actuator sleds (52, 54) as discussed above in detail as well as lower right and left actuator sleds (52, 54). As briefly discussed above, the description of upper right and left actuator sleds (52, 54) similarly applies to lower right and left actuator sleds (52, 54) with like features having like numbers, but with reversed transverse directions (e.g. lower, upward, etc.). To this end, upper left actuator sled (52) and lower right actuator sled (54) connect together as outer dowels (108) snap into outer dowel holes (92) and inner dowels (94) snap into inner dowel holes (106) with chassis (36) positioned therebetween. Upper right actuator sled (54) and lower left actuator sled (52) similarly connect together as outer dowels (108) snap into outer dowel holes (92) and inner dowels (94) snap into inner dowel holes (106) with chassis (36) positioned therebetween. Each of inner and outer dowels (94, 108) extend through sled clearance holes (70) to slidably connect left and right actuator sleds (52, 54) to chassis (36).

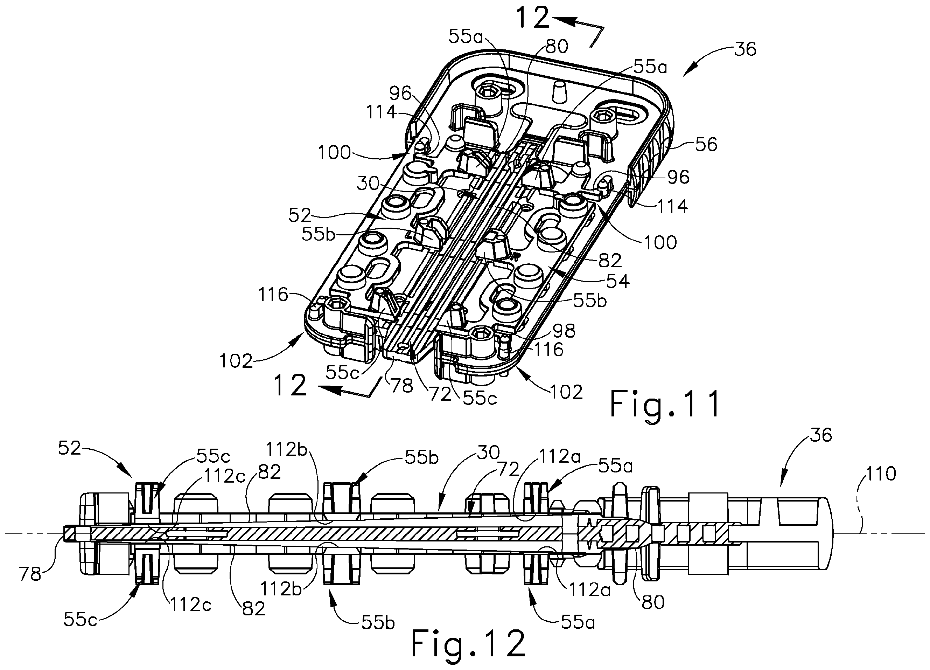

FIG. 12 shows one example of a pair of distal arms (55a), a pair of intermediate arms (55b), and a pair of proximal arms (55c) respectively having platform (30) positioned therebetween and tracing opposing ramp surfaces (82). A central plane (110) is shown in FIG. 12 bisecting upper and lower portions of buttress applier cartridge (16) through a central core of platform (30). Distal arm (55a) has a distal retention surface (112a) transversely offset from central plane (110) a relatively greater distance, intermediate arm (55b) has an intermediate retention surface (112b) transversely offset from central plane (110) a relatively intermediate distance, and proximal arm (55c) has a proximal retention surface (112c) transversely offset from central plane (110) a relatively lesser distance. Thereby, greater, intermediate, and lesser distances of distal, intermediate, and proximal retention surfaces (112a, 112b, 112c) trace ramp surfaces (82) tapering from distal end (80) of pad (72) to proximal end (78) of pad (72). Thus, distal, intermediate, and proximal retention surfaces (112a, 112b, 112c) are offset in the transverse direction from each other and from central plane (110). In the present example, each of distal arm (55a), intermediate arm (55b), and proximal arm (55c) are transversely spaced from the ramp surface (82) an equal transverse dimension such that arms (55a, 55b, 55c) equally trace ramp surfaces (82) tapering from distal end (80) of pad (72) to proximal end (78) of pad (72).

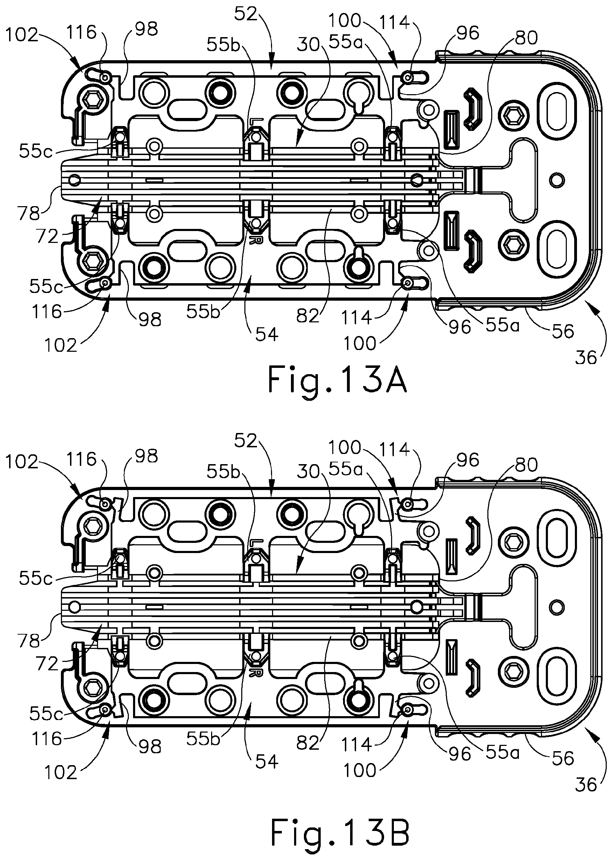

As shown in FIGS. 13A-13C, left and right actuator sleds (52, 54) are respectively urged outward from the restraint position to the release position away from platform to disengage arms (55a, 55b, 55c) from buttress assemblies (12) *(see* FIG. 1) on platform (30) as discussed herein. More particularly, distal and proximal detent couplings (100, 102) releasably connected left and right actuator sleds (52, 54) to chassis (36) to arrest movement of left and right actuator sleds (52, 54) in the restraint position and the release position. Distal and proximal detent couplings (100, 102) include distal and proximal cantilever catches (96, 98) extending from each of left and right actuator sleds (52, 54) as discussed briefly above. In addition, distal and proximal detent couplings (100, 102) respectively further include distal and proximal ground cams (114, 116) extending from chassis (36) in respective engagement with distal and proximal cantilever catches (96, 98).

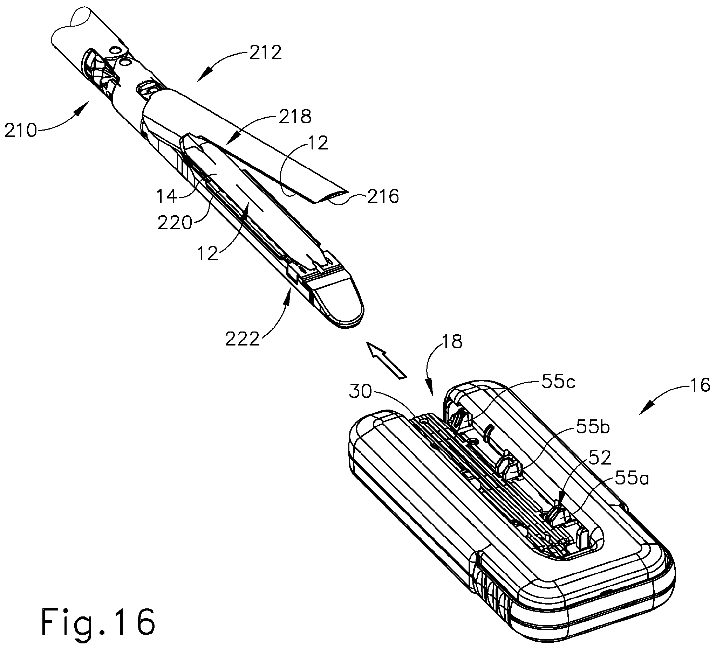

In the restraint position shown in FIG. 13A, each distal cantilever catch (96) is respectively engaged with each distal ground cam (114), and each proximal cantilever catch (98) is respectively engaged with each proximal ground cam (116) to urge left and right actuator sleds (52, 54) inward toward the restraint position. Directing left and right actuator sleds (52, 54) outward from the restraint position toward the release position as shown in FIG. 13B resiliently deflects distal and proximal cantilever catches (96, 98) as distal and proximal cantilever catches (96, 98) follow distal and proximal ground cams (114, 116). As distal and proximal cantilever catches (96, 98) pass around distal and proximal ground cams (114, 116), distal and proximal cantilever catches (96, 98) reach a tipping point where distal and proximal cantilever catches (96, 98) urge left and right actuator sleds (52, 54) to the release position shown in FIG. 13C. In the release position, each distal cantilever catch (96) is respectively engaged with each distal ground cam (114), and each proximal cantilever catch (98) is respectively engaged with each proximal ground cam (116) to urge left and right actuator sleds (52, 54) outward toward the release position. Thereby, distal and proximal detent couplings (100, 102) effectively hold left and right actuator sleds (52, 54) in the release position to inhibit arms (55a, 55b, 55c) from inadvertently returning inward and catching buttress assembly (12) *(see* FIG. 16) upon removal of end effector (212) *(see* FIG. 16) as discussed below in greater detail.

### C. Exemplary Adhesion of Buttress to Surgical Stapler and Cutting of Buttress Assembly with Tissue

As noted above and discussed below in greater detail with respect to FIG. 14A, upper and lower buttress assemblies (12) include upper and lower adhesive layers (42) (or other form of adhesive material) to adhere respective buttresses (14) to an underside (216) of anvil (218) and deck (220) of staple cartridge (222). Such adhesive may provide proper positioning of buttress (14) before and during actuation of end effector (212); then allow buttress (14) to separate from end effector (212) after end effector (212) has been actuated, without causing damage to buttress (14) that is substantial enough to compromise the proper subsequent functioning of buttress (14).

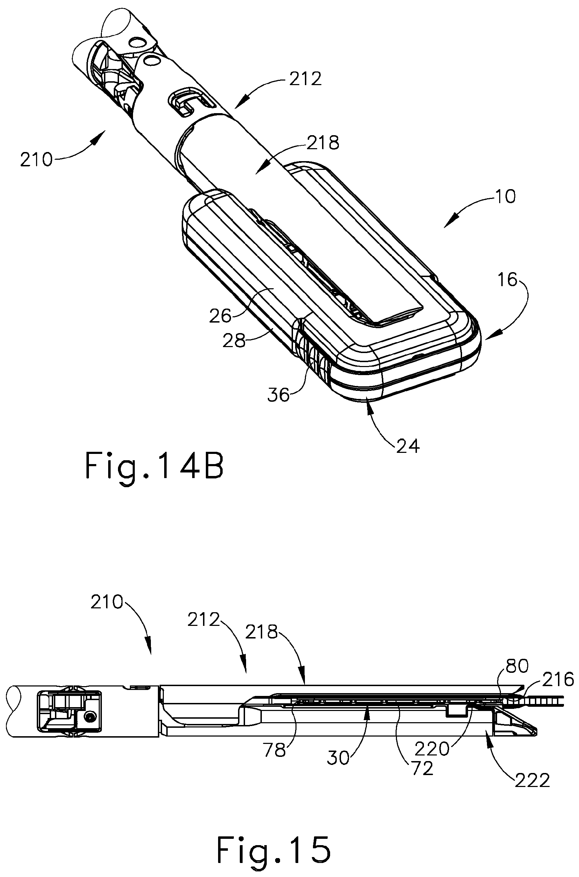

To use buttress applier cartridge (16) to load end effector (212), the operator would first position buttress applier cartridge (16) and end effector (212) such that end effector (212) is aligned with open end (18) of buttress applier cartridge (16) as shown in FIG. 14A. The operator would then advance end effector (212) distally (and/or retract buttress applier cartridge (16) proximally) to position platform (30) and buttress assemblies (12) between anvil (218) and staple cartridge (222). In order to load buttress assemblies (12) on end effector (212), the operator simply closes end effector (212) by pivoting anvil (218) toward staple cartridge (222) to reach the state shown in FIG. 14B.

As shown, closure of end effector (212) to the parallel-camber orientation results in anvil (218) and staple cartridge (222) bearing against actuator sleds (32), thereby urging arms (34) to unlock buttress assemblies (12) from buttress applier cartridge (16). Adhesive layers (42) of upper and lower buttress assemblies (12) are sufficiently compressed against anvil (218) and deck (220) to retain upper and lower buttress assemblies (12) to end effector (212) for stapling tissue. Pad (72) accommodates the parallel-camber orientation shown in FIG. 15 by providing reaction forces of at least the predetermined minimum force for adhesion along the longitudinal length of buttress assemblies (12). Upon depositing buttress assemblies (12) onto anvil (218) and staple cartridge (222), the operator removes buttress applier cartridge (16) from end effector (212) as shown in FIG. 16.

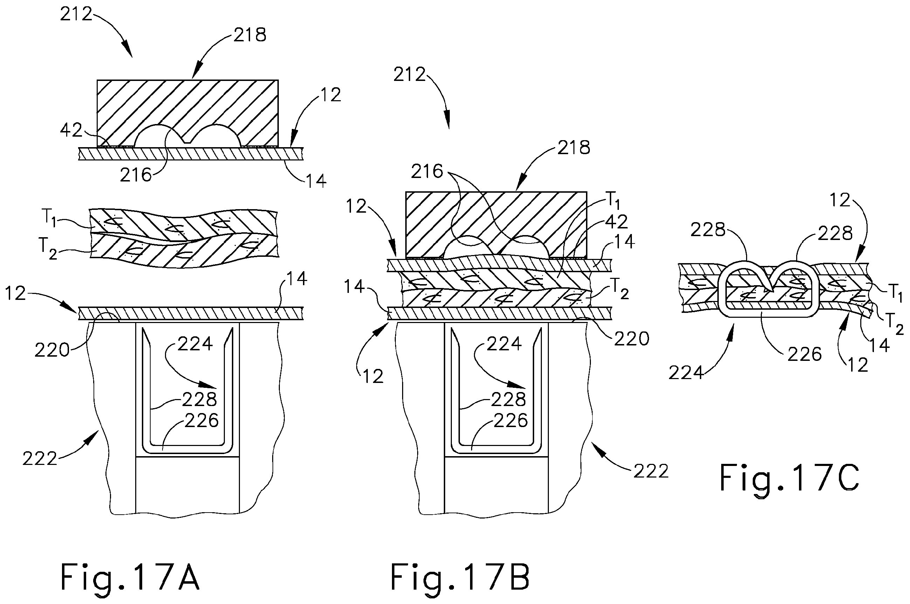

To this end, FIGS. 17A-17C show a sequence where end effector (212) loaded with buttress assemblies (12) is actuated to drive a plurality of staples (224) through two apposed layers of tissue (T₁, T₂), with buttress assemblies (12) being secured to the same layers of tissue (T₁, T₂) by staples (224). In particular, FIG. 17A shows layers of tissue (T₁, T₂) positioned between anvil (218) and staple cartridge (222), with anvil (218) in the open position. Upper buttress assembly (12) is adhered to the underside (216) of anvil (218) via adhesive layer (42); while lower buttress assembly (12) is adhered to deck (220) of staple cartridge (222) via adhesive layer (42). Layers of tissue (T₁, T₂) are thus interposed between upper and lower buttress assemblies (12). Next, a trigger (not shown) is pivoted to drive anvil (218) to the closed position as shown in FIG. 17B. At this stage, layers of tissue (T₁, T₂) are compressed between anvil (218) and staple cartridge (222), with upper and lower buttress assemblies (12) engaging opposite surfaces of tissue layers (T₁, T₂). End effector (212) is then actuated as described above, driving staple (224) through upper and lower buttress assemblies (12) and layers of tissue (T₁, T₂). As shown in FIG. 17C, a crown (226) of driven staple (224) captures and retains lower buttress assembly (12) against layer of tissue (T₂). Deformed legs (228) of staple (224) capture and retain upper buttress assembly (12) against layer of tissue (T₁).

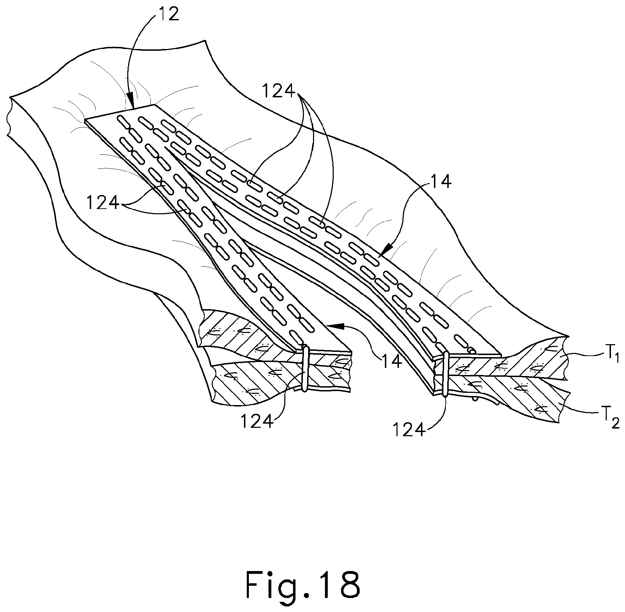

It should be understood that a series of staples (224) will similarly capture and retain upper and lower buttress assemblies (12) against layers of tissue (T₁, T₂), thereby securing upper and lower buttress assemblies (12) to tissue (T₁, T₂). In one example, knife (not shown) cuts through a centerline of buttress assemblies (12), separating buttress assemblies (12) into a corresponding pair of sections, such that each section remains secured to a respective severed region of tissue (T₁, T₂) as shown in FIG. 18.

With respect to FIG. 18, as end effector (212) *(see* FIG. 16) is pulled away from tissue (T₁, T₂) after deploying staples (224) and upper and lower buttress assemblies (12), upper and lower buttress assemblies (12) disengage end effector (212), such that upper and lower buttress assemblies (12) remain secured to tissue (T₁, T₂) with staples (224). Buttressed tissue (T₁, T₂) thus provides structural reinforcement to the lines of staples (224). In addition to the foregoing, it should also be understood that any of the various buttress assemblies described herein may be further constructed and operable in accordance with at least some of the teachings of U.S. Pat. Pub. No. 2016/0278774, entitled "Method of Applying a Buttress to a Surgical Stapler," published September 29, 2016.

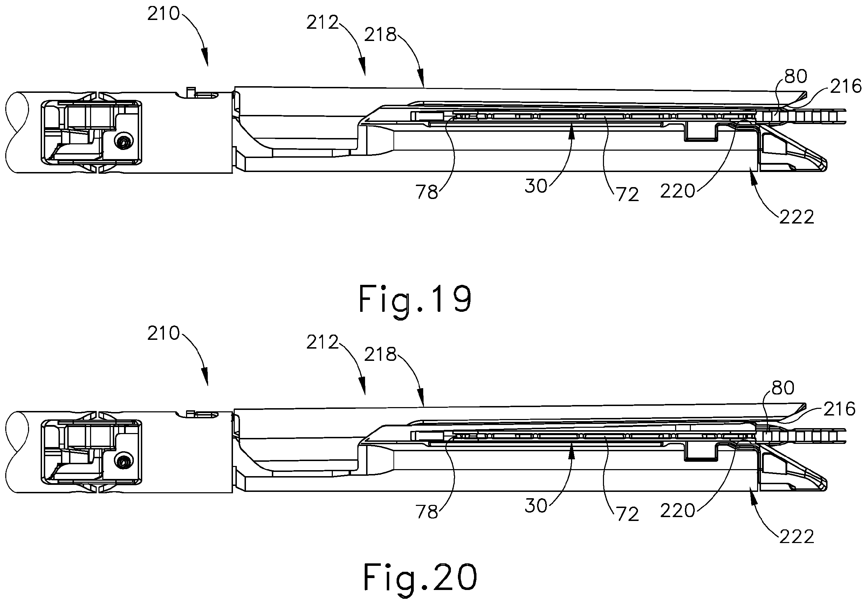

FIGS. 19-20 show pad (72) accommodating alternative camber orientations of end effector (212), such as the over-camber orientation and the under-camber orientation. With respect to FIG. 19, closure of end effector (212) to the over-camber orientation results in anvil (218) and staple cartridge (222) unlocking buttress assemblies (12) from buttress applier cartridge (16) as discussed above. Adhesive layers (42) of upper and lower buttress assemblies (12) are sufficiently compressed against anvil (218) and deck (220) to retain upper and lower buttress assemblies (12) to end effector (212) for stapling tissue. Pad (72) accommodates the over-camber orientation by providing reaction forces of at least the predetermined minimum force for adhesion along the longitudinal length of buttress assemblies (12).

Similarly, FIG. 20 shows closure of end effector (212) to the under-camber orientation resulting in anvil (218) and staple cartridge (222) unlocking buttress assemblies (12) from buttress applier cartridge (16) as discussed above. Adhesive layers (42) of upper and lower buttress assemblies (12) are sufficiently compressed against anvil (218) and deck (220) to retain upper and lower buttress assemblies (12) to end effector (212) for stapling tissue. Pad (72) accommodates the under-camber orientation by providing reaction forces of at least the predetermined minimum force for adhesion along the longitudinal length of buttress assemblies (12).

### III. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

**In** addition to the foregoing, it should also be understood that any of the various buttress assemblies described herein may be further constructed and operable in accordance with at least some of the teachings of U.S. Pat. Pub. No. 2016/0278774, entitled "Method of Applying a Buttress to a Surgical Stapler," published September 29, 2016; U.S. Pat. Pub. No. 2017/0049444, entitled "Implantable Layers for a Surgical Instrument," published February 23, 2017; U.S. Pat. Pub. No. 2017/0086837, entitled "Compressible Adjunct with Crossing Spacer Fibers," published March 30, 2017; and U.S. Pat. Pub. No. 2017/0086842, entitled "Method for Applying an Implantable Layer to a Fastener Cartridge," published March 30, 2017. Furthermore, in addition to the methods described herein, any of the various buttress assemblies described herein may be applied to end effector (212) in accordance with at least some of the teachings of U.S. Provisional Patent App. No. 62/209,041, entitled "Method and Apparatus for Applying a Buttress to End Effector of a Surgical Stapler," filed August 24, 2015; and/or U.S. Pat. Pub. No. 2017/0086842, entitled "Method for Applying an Implantable Layer to a Fastener Cartridge," published March 30, 2017. Various suitable ways in which the teachings herein may be combined with various teachings of the above-cited references will be apparent to those of ordinary skill in the art.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A buttress applier cartridge assembly (10), comprising:
(a) a first buttress assembly (12), including:
(i) a first buttress (14) configured to be received against tissue and support a staple formed therein, and
(ii) a first adhesive layer (42) on the first buttress (14) configured to releasably adhere the first buttress (14) against a first portion of an end effector (212) of a surgical stapler (210) upon engaging the first adhesive layer (42) against the end effector (212) with at least a predetermined minimum force;
(b) a buttress applier cartridge (16), including:
(i) a housing (24) defining a first gap (37) extending in a longitudinal direction therealong such that the first gap (37) is configured to receive the first portion of the end effector (212), and
(ii) a platform (30) extending longitudinally from a proximal platform portion (78) to a distal platform portion (80) and operatively connected to the housing (24), wherein the platform (30) is exposed in a transverse direction adjacent to the first gap (37), wherein the proximal and distal platform portions (78, 80) support the first buttress assembly (12) thereon;
(c) a first actuator sled (52) operatively connected to the housing (24) and configured to selectively move relative to the housing (24) from a first restraint position to a first release position, wherein the first actuator sled (52) in the first restraint position is configured to capture the first buttress assembly (12) to the platform (30), and wherein the first actuator sled (52) in the first release position is configured to release the first buttress assembly (12) from the platform (30) for removing the first buttress assembly (12) therefrom; and **characterized by**:
(d) a first detent coupling (100) operatively connected to the first actuator sled (52) and configured to arrest movement of the first actuator sled (52) in the restraint position when the first actuator sled (52) is in the first restraint position, and wherein the first detent coupling (100) is further configured to arrest movement of the first actuator sled (52) in the first release position when the first actuator sled (52) is in the first release position.

2. The buttress applier cartridge assembly of claim 1, further comprising:
(a) a second buttress assembly (12), including:
(i) a second buttress (14) configured to be received against tissue and support the staple formed therein, and
(ii) a second adhesive layer (42) on the second buttress (14) configured to releasably adhere the second buttress (14) against a second portion of the end effector (212) upon engaging the second adhesive layer (42) against the end effector (212) with at least the predetermined minimum force; and
(b) the buttress applier cartridge (16), further including:
(i) the housing (24) defining a second gap (38) extending in the longitudinal direction therealong such that the second gap (38) is configured to receive the second portion of the end effector (212), and
(ii) the platform (30) exposed in the transverse direction between the first and second gaps (37, 38),
wherein the proximal and distal platform portions (78, 80) support the first and second buttress assemblies (12) on opposing transverse sides thereof.

3. The buttress applier cartridge assembly of claim 1, further comprising:
(a) a second actuator sled (54) operatively connected to the housing (24) and configured to selectively move relative to the housing (24) from a second restraint position to a second release position, wherein the second actuator sled (54) in the second restraint position is configured to capture the first buttress assembly (12) to the platform (30), and wherein the second actuator sled (54) in the second release position is configured to release the first buttress assembly (12) from the platform (30) for removing the first buttress assembly (12) therefrom; and
(b) a second detent coupling (96) operatively connected to the second actuator sled (54) and configured to arrest movement of the second actuator sled (54) in the restraint position when the second actuator sled (54) is in the second restraint position, and wherein the second detent coupling (96) is further configured to arrest movement of the second actuator sled (54) in the second release position when the second actuator sled (54) is in the second release position.

4. The buttress applier cartridge assembly of claim 1, wherein the proximal platform portion (78) has a proximal transverse depth, wherein the distal platform portion (80) has a distal transverse depth, and wherein the proximal transverse depth (78) is different than the distal transverse depth (80), and wherein the first actuator sled (52) further includes a distal arm (55a) and a proximal arm (55c), wherein the distal arm (55a) extends laterally toward the first buttress assembly (12) and is longitudinally aligned with the distal platform portion (80), wherein the proximal arm (55c) extends laterally toward the first buttress assembly (12) and is longitudinally aligned with the proximal platform portion (78), and wherein the distal arm (55a) is transversely offset relative to the proximal arm (55c) such that the distal and proximal arms (55a, 55c) respectively engage the first buttress assembly (12) at the distal and proximal platform portions (78, 80) in the restraint position.

5. The buttress applier cartridge assembly of claim 1 or 4,
wherein the proximal and distal platform portions (78, 80) respectively have a proximal stiffness and a different, distal stiffness in the transverse direction, and
wherein the proximal and distal platform portions (78, 80) are configured to deform with the proximal and distal stiffnesses thereby providing reactionary forces of at least the predetermined minimum force while receiving the end effector (212) for accommodating various camber orientations of the end effector (212).

## Patentansprüche

1. Versteifungsapplikatorpatronenanordnung (10), umfassend:
(a) eine erste Versteifungsanordnung (12), einschließlich:
(i) einer ersten Versteifung (14), die konfiguriert ist, um gegen Gewebe aufgenommen zu werden und eine darin geformte Klammer zu stützen, und
(ii) einer ersten Klebeschicht (42) auf der ersten Versteifung (14), die konfiguriert ist, um die erste Versteifung (14) lösbar an einem ersten Abschnitt eines Endeffektors (212) einer chirurgischen Klammervorrichtung (210) beim Ineingriffbringen der ersten Klebeschicht (42) gegen den Endeffektor (212) mit mindestens einer vorgegebenen Mindestkraft zu halten;
(b) eine Versteifungsapplikatorpatrone (16), einschließlich:
(i) eines Gehäuses (24), das einen ersten Spalt (37) definiert, der sich daran in einer Längsrichtung entlang auf derartige Weise erstreckt, dass der erste Spalt (37) konfiguriert ist, um den ersten Abschnitt des Endeffektors (212) aufzunehmen, und
(ii) einer Plattform (30), die sich in Längsrichtung von einem proximalen Plattformabschnitt (78) zu einem distalen Plattformabschnitt (80) erstreckt und betreibbar mit dem Gehäuse (24) verbunden ist, wobei die Plattform (30) in einer Querrichtung neben dem ersten Spalt (37) freiliegt,
wobei der proximale und der distale Plattformabschnitt (78, 80) die erste Versteifungsanordnung (12) darauf stützen;
(c) einen ersten Aktuatorschlitten (52), der betreibbar mit dem Gehäuse (24) verbunden ist und konfiguriert ist, um sich in Bezug auf das Gehäuse (24) selektiv von einer ersten Rückhalteposition in eine erste Freigabeposition zu bewegen, wobei der erste Aktuatorschlitten (52) in der ersten Rückhalteposition konfiguriert ist, um die erste Versteifungsanordnung (12) an der Plattform (30) festzuhalten, und wobei der erste Aktuatorschlitten (52) in der ersten Freigabeposition konfiguriert ist, um die erste Versteifungsanordnung (12) von der Plattform (30) freizugeben, um die erste Versteifungsanordnung (12) davon zu entfernen; und **gekennzeichnet durch:**
(d) eine erste Rastkopplung (100), die betreibbar mit dem ersten Aktuatorschlitten (52) verbunden ist und konfiguriert ist, um die Bewegung des ersten Aktuatorschlittens (52) in der Rückhalteposition zu stoppen, wenn sich der erste Aktuatorschlitten (52) in der ersten Rückhalteposition befindet, und wobei die erste Rastkopplung (100) ferner konfiguriert ist, um die Bewegung des ersten Aktuatorschlittens (52) in der ersten Freigabeposition zu stoppen, wenn sich der erste Aktuatorschlitten (52) in der ersten Freigabeposition befindet.

2. Versteifungsapplikatorpatronenanordnung nach Anspruch 1, ferner umfassend:
(a) eine zweite Versteifungsanordnung (12), einschließlich:
(i) einer zweiten Versteifung (14), die konfiguriert ist, um gegen Gewebe aufgenommen zu werden und die darin geformte Klammer zu stützen, und
(ii) einer zweiten Klebeschicht (42) auf der zweiten Versteifung (14), die konfiguriert ist, um die zweite Versteifung (14) lösbar gegen einen zweiten Abschnitt des Endeffektors (212) beim Ineingriffbringen der zweiten Klebeschicht (42) gegen den Endeffektor (212) mit mindestens der vorgegebenen Mindestkraft zu halten; und
(b) die Versteifungsapplikatorpatrone (16), ferner einschließlich:
(i) des Gehäuses (24), das einen zweiten Spalt (38) definiert, der sich daran in der Längsrichtung entlang auf derartige Weise erstreckt, dass der zweite Spalt (38) konfiguriert ist, um den zweiten Abschnitt des Endeffektors (212) aufzunehmen, und
(ii) der Plattform (30), die in Querrichtung zwischen dem ersten und dem zweiten Spalt (37, 38) freiliegt,
wobei der proximale und der distale Plattformabschnitt (78, 80) die erste und die zweite Versteifungsanordnung (12) an gegenüberliegenden Querseiten davon stützen.

3. Versteifungsapplikatorpatronenanordnung nach Anspruch 1, ferner umfassend:
(a) einen zweiten Aktuatorschlitten (54), der betreibbar mit dem Gehäuse (24) verbunden ist und konfiguriert ist, um sich in Bezug auf das Gehäuse (24) selektiv von einer zweiten Rückhalteposition in eine zweite Freigabeposition zu bewegen, wobei der zweite Aktuatorschlitten (54) in der zweiten Rückhalteposition konfiguriert ist, um die erste Versteifungsanordnung (12) an der Plattform (30) festzuhalten, und wobei der zweite Aktuatorschlitten (54) in der zweiten Freigabeposition konfiguriert ist, um die erste Versteifungsanordnung (12) von der Plattform (30) freizugeben, um die erste Versteifungsanordnung (12) davon zu entfernen; und
(b) eine zweite Rastkopplung (96), die betreibbar mit dem zweiten Aktuatorschlitten (54) verbunden ist und konfiguriert ist, um die Bewegung des zweiten Aktuatorschlittens (54) in der Rückhalteposition zu stoppen, wenn sich der zweite Aktuatorschlitten (54) in der zweiten Rückhalteposition befindet, und wobei die zweite Rastkopplung (96) ferner konfiguriert ist, um die Bewegung des zweiten Aktuatorschlittens (54) in der zweiten Freigabeposition zu stoppen, wenn sich der zweite Aktuatorschlitten (54) in der zweiten Freigabeposition befindet.

4. Versteifungsapplikatorpatronenanordnung nach Anspruch 1, wobei der proximale Plattformabschnitt (78) eine proximale Quertiefe aufweist, wobei der distale Plattformabschnitt (80) eine distale Quertiefe aufweist, und wobei die proximale Quertiefe (78) verschieden von der distalen Quertiefe (80) ist, und wobei der erste Betätigungsschlitten (52) ferner einen distalen Arm (55a) und einen proximalen Arm (55c) einschließt, wobei sich der distale Arm (55a) seitlich in Richtung der ersten Versteifungsanordnung (12) erstreckt und in Längsrichtung auf den distalen Plattformabschnitt (80) ausgerichtet ist, wobei sich der proximale Arm (55c) seitlich in Richtung der ersten Versteifungsanordnung (12) erstreckt und in Längsrichtung auf den proximalen Plattformabschnitt (78) ausgerichtet ist, und wobei der distale Arm (55a) relativ zu dem proximalen Arm (55c) quer versetzt ist, sodass der distale und der proximale Arm (55a, 55c) in der Rückhalteposition jeweils an dem distalen und dem proximalen Plattformabschnitt (78, 80) die erste Versteifungsanordnung (12) in Eingriff bringen.

5. Versteifungsapplikatorpatronenanordnung nach Anspruch 1 oder 4,
wobei der proximale und der distale Plattformabschnitt (78, 80) jeweils eine proximale Steifigkeit und eine unterschiedliche, distale Steifigkeit in der Querrichtung aufweisen, und
wobei der proximale und der distale Plattformabschnitt (78, 80) konfiguriert sind, um sich mit der proximalen und distalen Steifheit zu verformen, wodurch Reaktionskräfte von mindestens der vorgegebenen Mindestkraft bereitgestellt werden, während der Endeffektor (212) zum Ausgleichen verschiedener Wölbungsausrichtungen des Endeffektors (212) aufgenommen wird.

## Revendications

1. Ensemble cartouche d'applicateur de contrefort (10), comprenant :
(a) un premier ensemble contrefort (12), comportant :
(i) un premier contrefort (14) conçu pour être reçu contre un tissu et supporter une agrafe formée dans celui-ci, et
(ii) une première couche adhésive (42) sur le premier contrefort (14) conçue pour faire adhérer de manière libérable le premier contrefort (14) contre une première partie d'un effecteur terminal (212) d'une agrafeuse chirurgicale (210) lors d'une mise en prise de la première couche adhésive (42) contre l'effecteur terminal (212) avec au moins une force minimale prédéterminée ;
(b) une cartouche d'applicateur de contrefort (16), comportant :
(i) un logement (24) définissant un premier espace (37) s'étendant dans une direction longitudinale le long de celui-ci de telle sorte que le premier espace (37) est conçu pour recevoir la première partie de l'effecteur terminal (212), et
(ii) une plate-forme (30) s'étendant longitudinalement à partir d'une partie de plate-forme proximale (78) jusqu'à une partie de plate-forme distale (80) et reliée fonctionnellement au logement (24), dans lequel la plate-forme (30) est exposée dans une direction transversale adjacente au premier espace (37),
dans lequel les parties de plate-forme proximale et distale (78, 80) supportent le premier ensemble contrefort (12) sur celles-ci ;
(c) un premier traîneau actionneur (52) relié fonctionnellement au logement (24) et conçu pour se déplacer sélectivement par rapport au logement (24) d'une première position de retenue à une première position de libération, dans lequel le premier traîneau actionneur (52) dans la première position de retenue est conçu pour capturer le premier ensemble contrefort (12) sur la plate-forme (30), et dans lequel le premier traîneau actionneur (52) dans la première position de libération est conçu pour libérer le premier ensemble contrefort (12) de la plate-forme (30) pour retirer le premier ensemble contrefort (12) de celle-ci ; et **caractérisé par** :
(d) un premier accouplement à crans (100) relié fonctionnellement au premier traîneau actionneur (52) et conçu pour arrêter un déplacement du premier traîneau actionneur (52) dans la position de retenue lorsque le premier traîneau actionneur (52) est dans la première position de retenue, et dans lequel le premier accouplement à crans (100) est configuré en outre pour arrêter un déplacement du premier traîneau actionneur (52) dans la première position de libération lorsque le premier traîneau actionneur (52) est dans la première position de libération.

2. Ensemble cartouche d'applicateur de contrefort selon la revendication 1, comprenant en outre :
(a) un second ensemble contrefort (12), comportant :
(i) un second contrefort (14) conçu pour être reçu contre un tissu et supporter l'agrafe formée dans celui-ci, et
(ii) une seconde couche adhésive (42) sur le second contrefort (14) conçue pour faire adhérer de manière libérable le second contrefort (14) contre une seconde partie de l'effecteur terminal (212) lors d'une mise en prise de la seconde couche adhésive (42) contre l'effecteur terminal (212) avec au moins la force minimale prédéterminée ; et
(b) la cartouche d'applicateur de contrefort (16), comportant en outre :
(i) le logement (24) définissant un second espace (38) s'étendant dans la direction longitudinale le long de celui-ci de telle sorte que le second espace (38) est conçu pour recevoir la seconde partie de l'effecteur terminal (212), et
(ii) la plate-forme (30) exposée dans la direction transversale entre les premier et second espaces (37, 38),
dans lequel les parties de plate-forme proximale et distale (78, 80) supportent les premier et second ensembles contreforts (12) sur des côtés transversaux opposés de ceux-ci.

3. Ensemble cartouche d'applicateur de contrefort selon la revendication 1, comprenant en outre :
(a) un second traîneau actionneur (54) relié fonctionnellement au logement (24) et conçu pour se déplacer sélectivement par rapport au logement (24) d'une seconde position de retenue à une seconde position de libération, dans lequel le second traîneau actionneur (54) dans la seconde position de retenue est conçu pour capturer le premier ensemble contrefort (12) sur la plate-forme (30), et dans lequel le second traîneau actionneur (54) dans la seconde position de libération est conçu pour libérer le premier ensemble contrefort (12) de la plate-forme (30) pour retirer le premier ensemble contrefort (12) de celle-ci ; et
(b) un second accouplement à crans (96) relié fonctionnellement au second traîneau actionneur (54) et conçu pour arrêter un déplacement du second traîneau actionneur (54) dans la position de retenue lorsque le second traîneau actionneur (54) est dans la seconde position de retenue, et dans lequel le second accouplement à crans (96) est conçu en outre pour arrêter un déplacement du second traîneau actionneur (54) dans la seconde position de libération lorsque le second traîneau actionneur (54) est dans la seconde position de libération.

4. Ensemble cartouche d'applicateur de contrefort selon la revendication 1, dans lequel la partie de plate-forme proximale (78) a une profondeur transversale proximale, dans lequel la partie de plate-forme distale (80) a une profondeur transversale distale, et dans lequel la profondeur transversale proximale (78) est différente de la profondeur transversale distale (80), et dans lequel le premier traîneau actionneur (52) comporte en outre un bras distal (55a) et un bras proximal (55c), dans lequel le bras distal (55a) s'étend latéralement en direction du premier ensemble contrefort (12) et est longitudinalement aligné avec la partie de plate-forme distale (80), dans lequel le bras proximal (55c) s'étend latéralement en direction du premier ensemble contrefort (12) et est longitudinalement aligné avec la partie de plate-forme proximale (78), et dans lequel le bras distal (55a) est transversalement décalé par rapport au bras proximal (55c) de telle sorte que les bras distal et proximal (55a, 55c) viennent respectivement en prise avec le premier ensemble contrefort (12) au niveau des parties de plate-forme distale et proximale (78, 80) dans la position de retenue.

5. Ensemble cartouche d'applicateur de contrefort selon la revendication 1 ou 4,
dans lequel les parties de plate-forme proximale et distale (78, 80) ont respectivement une rigidité proximale et une rigidité distale, différente, dans la direction transversale, et
dans lequel les parties de plate-forme proximale et distale (78, 80) sont conçues pour se déformer avec les rigidités proximale et distale en fournissant de ce fait des forces de réaction d'au moins la force minimale prédéterminée tout en recevant l'effecteur terminal (212) pour s'adapter à diverses orientations de cambrure de l'effecteur terminal (212).
